# DEMANDE DE BREVET EUROPEEN

(11) **EP 0 803 505 A1**
(43) Date de publication de la demande: **29.10.1997**
(21) Numéro de dépôt: 97400913.6
(22) Date de dépôt: 23.04.1997
(51) Int. Cl.: C07D 495/04, C07D 471/04, C07D 221/10, C07D 217/26, C07D 209/42, C07D 493/04, A61K 31/435, A61K 31/47, A61K 31/415, A61K 31/40

(54) **Nouveaux inhibiteurs de métalloprotéases, leur procédé de préparation et les compositions pharmaceutiques qui les contiennent**

(30) Priorité: 26.04.1996 FR 9605321
(71) Demandeur: ADIR ET COMPAGNIE, 92415 Courbevoie Cédex (FR)
(72) Inventeur: de Nanteuil, Guillaume, 92150 Suresnes (FR); Paladino, Joseph, 78700 Conflans Sainte Honorine (FR); Remond, Georges, 78000 Versailles (FR); Atassi, Ghanem, 92210 Saint Cloud (FR); Pierre, Alain, 78580 Les Alluets le Roi (FR); Tucker, Gordon, 75017 Paris (FR); Bonnet, Jacqueline, 75013 Paris (FR); Sabatini, Massimo, 92380 Garches (FR)

(57) **Abrégé**

Composé de formule (I) : 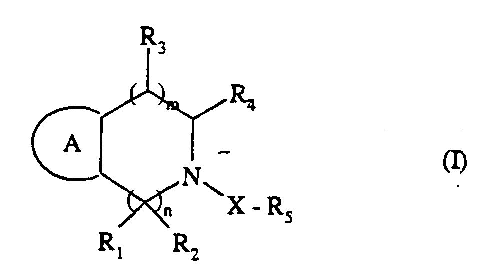 dans laquelle :
- m, n,: identiques ou différents, représentent 0, 1 ou 2,
- R₁, R₂,: identiques ou différents, représentent un atome d'hydrogène, un groupement alkyle éventuellement substitué, un groupement aryle, ou forment avec l'atome de carbone qui les porte un groupement carbonyle ou un groupement cycloalkyle (C₃-C₇),
- R₃: représente un atome d'hydrogène, un groupement alkyle, hydroxy ou un groupement aryle,
- R₄: représente l'un quelconque des groupements suivants : 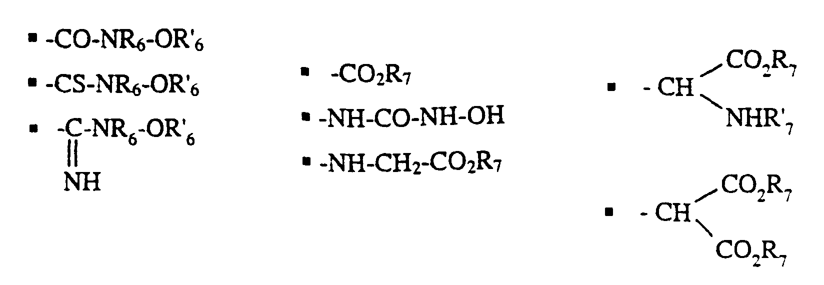
- X: représente -SO₂-, -CO- ou -SO₂NH-,
- R₅: représente un groupement alkyle, éventuellement substitué, cycloalkyle (C₃-C₇), aryle ou hétérocyclique,
- A: représente un cycle aryle ou un hétérocycle,
leurs isomères ainsi que leurs sels d'addition à un acide ou à une base pharmaceutiquement acceptable.

## Description

La présente invention concerne de nouveaux inhibiteurs de métalloprotéases, leur procédé de préparation et les compositions pharmaceutiques qui les contiennent.

A l'état physiologique, la synthèse des tissus connectifs est en équilibre dynamique avec la dégradation de la matrice extracellulaire. Cette dégradation est dûe à des protéases à zinc (métalloprotéases) secrétées par les cellules de la matrice existante : ce sont de façon non limitative les collagénases (MMP-1), les gélatinases ou collagénases de type IV (MMP-2, MMP-9) et les stromélysines (MMP-3).

A l'état normal, ces enzymes cataboliques sont régulées au niveau de leur synthèse et de leur sécrétion, ainsi qu'au niveau de leur activité enzymatique extracellulaire par des inhibiteurs naturels, comme l'α₂-macroglobuline ou les TIMP (Tissue Inhibitor of MetalloProteinase) qui forment des complexes inactifs avec les métalloprotéases.

Le point commun aux pathologies impliquant ces enzymes est un déséquilibre entre l'activité des enzymes activées et celle de leurs inhibiteurs naturels, avec pour conséquence une dégradation excessive des tissus.

La dégradation incontrôlée et accélérée des membranes par la résorption de la matrice extracellulaire catalysée par les métalloprotéases est un paramètre commun à plusieurs conditions pathologiques comme l'arthrite rhumatoïde, l'arthrose, l'invasion et la croissance tumorale, y compris la dissémination maligne et la formation de métastases, les ulcérations, l'athérosclérose, etc...

Récemment, le BB94, inhibiteur de métalloprotéases a montré une activité antitumorale en clinique où il s'est révélé actif sur les cancers de l'ovaire (Becket et al., DDT 1996, 1 (1), 16).

On peut donc attendre d'un inhibiteur de métalloprotéases qu'il restaure l'équilibre entre protéase et inhibiteur et de ce fait modifie de façon favorable l'évolution de ces pathologies.

Un certain nombre d'inhibiteurs de métalloprotéases ont été décrits dans la littérature. C'est le cas, plus particulièrement, des composés décrits dans les brevets WO 95/35275, WO 95/35276, EP 606046 ou WO 96/00214.

Les composés de la présente invention, outre le fait qu'ils soient nouveaux, se sont révélés être des inhibiteurs de métalloprotéases plus puissants que ceux décrits dans la littérature ce qui les rend donc potentiellement utiles pour le traitement des cancers, des maladies rhumatismales comme l'arthrose et l'arthrite rhumatoïde, de l'athérosclérose, etc...

Plus spécifiquement, la présente invention concerne les composés de formule (I) : 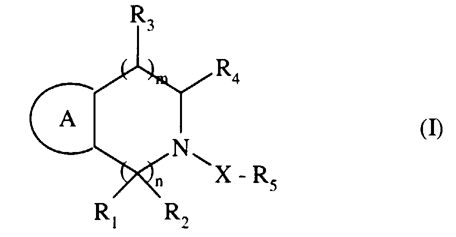 dans laquelle :
- m, n,: identiques ou différents, représentent 0, 1 ou 2,
- R₁, R₂,: identiques ou différents, représentent un atome d'hydrogène, un groupement alkyle (C₁-C₆) linéaire ou ramifié (éventuellement substitué par un groupement aryle), un groupement aryle, ou forment avec l'atome de carbone qui les porte un groupement carbonyle ou un groupement cycloalkyle (C₃-C₇),
- R₃: représente un atome d'hydrogène, un groupement alkyle (C₁-C₆) linéaire ou ramifié, hydroxy, alkoxy (C₁-C₆) linéaire ou ramifié ou un groupement aryle,
- R₄: représente l'un quelconque des groupements suivants :

■ -CO-NR₆-OR'₆
■ -CS-NR₆-OR'₆
■ 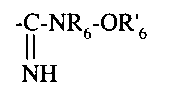 dans lesquels :
   R₆ et R'₆, identiques ou différents, représentent un atome d'hydrogène ou un groupement alkyle (C₁-C₆) linéaire ou ramifié,
■ -CO₂R₇
■ -NH-CO-NH-OH
■ -NH-CH₂-CO₂R₇
■ 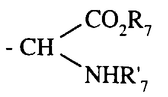
■ 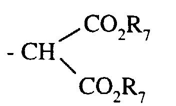 dans lesquels :
   R₇, R'₇, identiques ou différents, représentent un atome d'hydrogène ou un groupement alkyle (C₁-C₆) linéaire ou ramifié (éventuellement substitué par un groupement aryle),

- X: représente -SO₂-, -CO- ou -SO₂NH-,
- R₅: représente :

- un groupement alkyle (C₁-C₆) linéaire ou ramifié, éventuellement substitué par un ou plusieurs atomes d'halogène ou groupements hydroxy, alkoxy (C₁-C₆) linéaire ou ramifié, aryle ou -CO₂R₇ (dans lequel R₇ a la même signification que précédemment),
- un groupement cycloalkyle (C₃-C₇),
- un groupement aryle,
- ou, un groupement hétérocyclique,

- A: représente un cycle aryle (à la condition que ce cycle aryle soit différent d'un cycle phényle lorsque X représente SO₂, m et n représentent simultanément le nombre 1, R₄ représente -CO-NHOH, et R₅ représente un groupement aryle ou un groupement hétérocyclique), ou un hétérocycle,
leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle, on entend phényle, naphtyle, tétrahydronaphtyle, chacun de ces groupements étant éventuellement substitué par un ou plusieurs atomes d'halogène ou groupement alkyle (C₁-C₆) linéaire ou ramifié, trihalogénoalkyle (C₁-C₆) linéaire ou ramifié, cycloalkyle (C₃-C₇), bicycloalkyle (C₅-C₁₀), phényle éventuellement substitué, pyridyle éventuellement substitué, pyrimidyle éventuellement substitué, alkoxy (C₁-C₆) linéaire ou ramifié (éventuellement substitué par un groupement amino, lui-même éventuellement substitué par un ou deux groupements alkyle), trihalogénoalkoxy (C₁-C₆) linéaire ou ramifié, hydroxy, cyano ou amino (substitué éventuellement par un ou plusieurs groupements alkyle (C₁-C₆) linéaire ou ramifié).

Par hétérocycle, on entend un groupement mono ou bicyclique, saturé ou insaturé, de 5 à 16 chaînons contenant un, deux ou trois hétéroatomes choisis parmi oxygène, azote ou soufre, étant entendu que l'hétérocycle peut être éventuellement substitué par un ou plusieurs atomes d'halogène ou groupements alkyle (C₁-C₆) linéaire ou ramifié (éventuellement substitué par un groupement aryle), alkoxy (C₁-C₆) linéaire ou ramifié, hydroxy, trihalogénométhyle ou amino (substitué éventuellement par un ou plusieurs groupements alkyle (C₁-C₆) linéaire ou ramifié).

Les composés préférés selon l'invention sont ceux pour lesquels :
A représente un cycle aryle choisi parmi phényle ou naphtyle, substitués ou non, ou un hétérocycle choisi parmi les cycles thiophène, indole, furane, benzo[b]thiophène, imidazole, pyridine, benzofurane, pyrrole, quinoléine, substitués ou non.

Les composés préférés de l'invention sont ceux pour lesquels X représente SO₂, m = 1, n = 1, R₁, R₂, R₃ représentent simultanément un atome d'hydrogène.

Les substituants R₄ préférés selon l'invention sont les groupements -CO-NR₆-OR'₆.

Les substituants R₅ préférés sont les groupements aryle ou les groupements hétérocycliques et plus préférentiellement les groupements phényles substitués ou non, naphtyles substitués ou non ou pyridyles substitués ou non.

L'invention s'étend également au procédé de préparation des composés de formule (I), caractérisé en ce que l'on utilise comme produit de départ un acide de formule (II), sous forme racémique ou d'isomère défini : 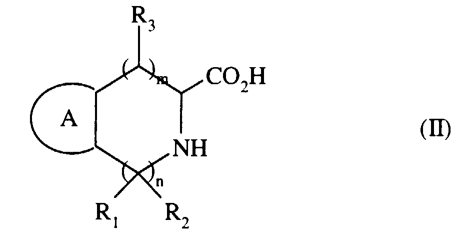 dans laquelle R₁, R₂, R₃, m, n et A ont la même signification que dans la formule (I), dont on substitue la fonction amine par un dérivé halogéné de formule (III) :

R₅ - X- Hal (III)

dans laquelle X et R₅ ont la même signification que dans la formule (I) et Hal représente un atome d'halogène,
pour conduire au composé de formule (I/a), cas particulier des composés de formule (I) : 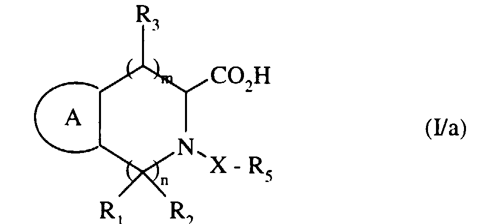 dans laquelle A, R₁, R₂, R₃, R₅, X, m et n ont la même signification que dans la formule (I),
composé de formule (I/a):
- (a) dont on transforme, si on le souhaite, la fonction acide en fonction ester correspondante,
- (b) ou, que l'on met en réaction avec une hydroxylamine-O- substituée
   pour conduire, après déprotection de la fonction hydroxylamine et substitution éventuelle de la fonction hydroxylamine, au composé de formule (I/b), cas particulier des composés de formule (I) : 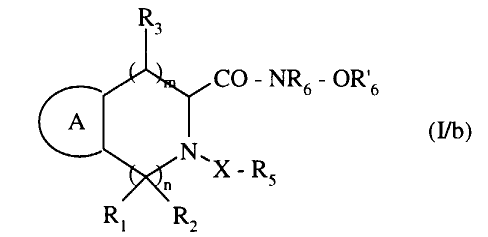 dans laquelle A, R₁, R₂, R₃, R₅, R₆, R'₆, X, m et n ont la même signification que dans la formule (I),
   - composé de formule (I/b) que l'on soumet, si on le désire, à l'action du réactif de Lawesson dans l'orthoxylène,
      pour conduire au composé de formule (I/c), cas particulier des composés de formule (I) : 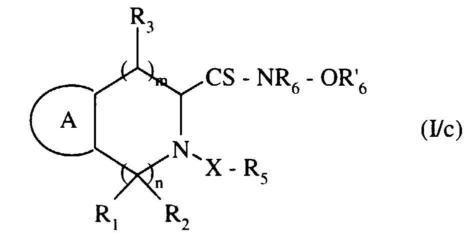 dans laquelle A, R₁, R₂, R₃, R₅, R₆, R'₆, X, m et n ont la même signification que dans la formule (I),
   - composé de formule (I/c), que l'on soumet, si on le souhaite, à l'action de l'ammoniaque, pour conduire au composé de formule (I/d), cas particulier des composés de formule (I) : 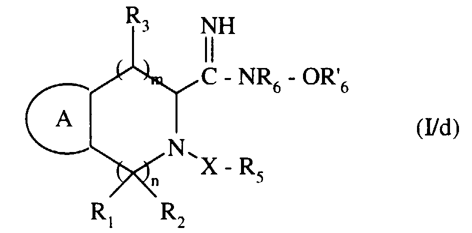 dans laquelle A, R₁, R₂, R₃, R₅, R₆, R'₆, X, m et n ont la même signification que dans la formule (I),
- (c) que l'on transforme en amine primaire de formule (IV) : 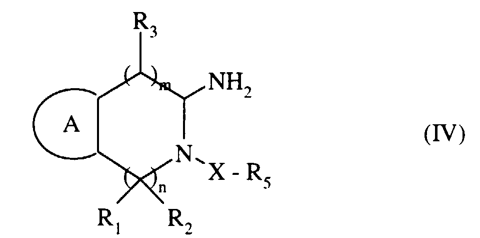 dans laquelle A, R₁, R₂, R₃, R₅, X, m et n ont la même signification que dans la formule (I), que l'on met en réaction :
   - soit avec le carbonyldiimidazole et une hydroxylamine protégée,
      pour conduire au composé de formule (I/e), cas particulier des composés de formule (I) : 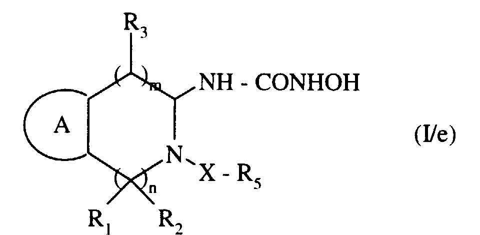 dans laquelle A, R₁, R₂, R₃, R₅, X, m et n ont la même signification que dans la formule (I),
   - soit avec un bromoacétate, pour conduire au composé de formule (I/f), cas particulier des composés de formule (I) : 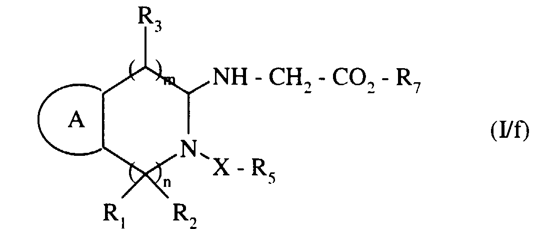 dans laquelle A, R₁, R₂, R₃, R₅, R₇, X, m et n ont la même signification que dans la formule (I),
- (d) dont on réduit l'acide en forme alcool correspondante, puis que l'on transforme en dérivé bromé par action de PBr₃ dans l'éther,
   pour conduire au composé de formule (V) : 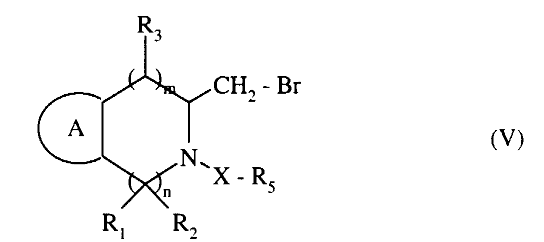 dans laquelle A, R₁, R₂, R₃, R₅, X, m et n ont la même signification que dans la formule (I), que l'on met en réaction :
   - soit avec un malonate d'alkyle,
      pour conduire, après saponification éventuelle, au composé de formule (I/g) : 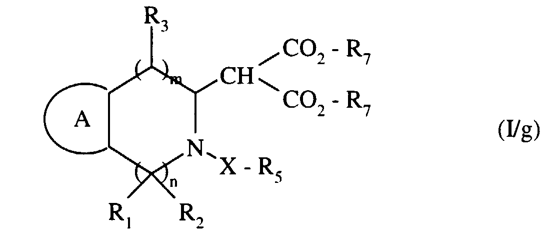 dans laquelle A, R₁, R₂, R₃, R₅, R₇, X, m et n ont la même signification que dans la formule (I),
   - soit avec une glycine protégée,
      pour conduire, après déprotection éventuelle, au composé de formule (I/h) : 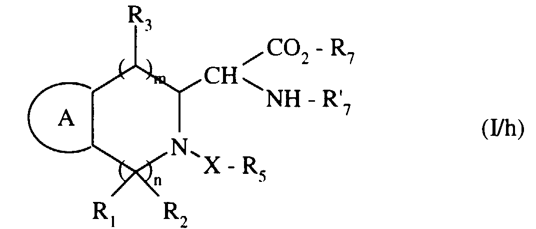 dans laquelle A, R₁, R₂, R₃, R₅, R₇, R'₇, X, m et n ont la même signification que dans la formule (I),
composé de formule (I/a) à (I/h), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

Les composés de formule (II) sont, soit des composés commerciaux, soit obtenus, lorsque n = 1, par cyclisation d'un acide aminé libre de formule (VI) : 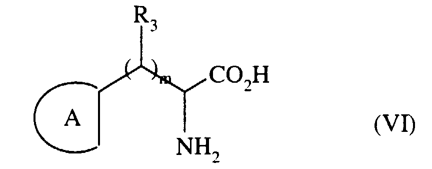 dans laquelle A, R₃ et n ont la même signification que dans la formule (I), avec une cétone de formule R₁R₂CO dans laquelle R₁ et R₂ ont la même signification que dans la formule (I).

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 0,01 à 2 g par jour en une ou plusieurs prises.

Les exemples suivants illustrent l'invention mais ne la limitent en aucune façon.

Les produits de départ utilisés sont des produits connus ou préparés selon des modes opératoires connus.

Les préparations conduisent à des intermédiaires de synthèse, utiles pour la préparation des composés de l'invention.

Les structures des composés décrits dans les exemples et les préparations ont été déterminées selon les techniques spectrophotométriques usuelles (infrarouge, RMN, spectrométrie de masse, ...).

### Préparation A : Acide 4,5,6,7-tétrahydrothiéno[3,2-c]pyridine-(6R)-carboxylique, chlorhydrate

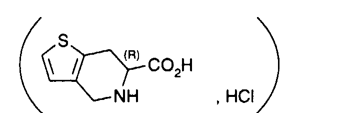

Un mélange contenant 87 mmoles de β-2-thiényl-D-alanine, 11 ml de formaldéhyde à 40 % en milieu aqueux, et 88 ml d'acide chlorhydrique 1N est chauffé 2 h 30 à 110°C sous agitation, puis à 60°C pendant une nuit. Après évaporation, le résidu est repris à l'éthanol puis évaporé et le produit attendu est récupéré sous forme solide dans de l'éther diéthylique.
*Point de fusion : 260°C*
*Microanalyse élémentaire :*

| | *C %* | *H %* | *N %* | *S %* | *Cl %* |
|---|---|---|---|---|---|
| *calculé* | *43,74* | *4,59* | *6,38* | *14,60* | *16,14* |
| *trouvé* | *43,68* | *4,72* | *6,45* | *14,54* | *15,68* |

### Préparation B : Acide 1,2,3,4-tétrahydro-β-carboline-(3R)-carboxylique

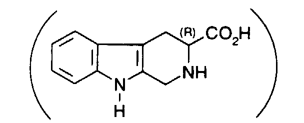

Le produit attendu est synthétisé selon le procédé décrit par L. TILSTRA et coll., (J. Am. Chem. Soc., 112, 9176-9182, 1990) à partir de (D)-tryptophane.

### Préparation C : Acide 1,2,3,4-tétrahydrobenzo[b]thiéno[2,3-c]pyridine-(3R)-carboxylique, chlorhydrate

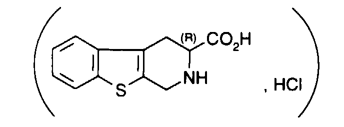

Le produit attendu est obtenu selon le procédé décrit dans la préparation A à partir de β-3-benzo[b]thiényl-D-alanine
*Point de fusion :* > *260°C*

### Préparation D : Acide 1,2,3,4,tétrahydrobenzo[h]isoquinoléine-(3R)-carboxylique, chlorhydrate

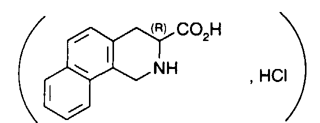

Le produit attendu est obtenu selon le procédé décrit dans la préparation A à partir de β-2-naphtyl-D-alanine en utilisant de l'acide chlorhydrique concentré au lieu de l'acide chlorhydrique 1N.
*Point de fusion : > 260°C*

### Préparation E : Acide 1,2,3,4-thétrahydrobenzo[f]isoquinoléine-(3R)- carboxylique, chlorhydrate

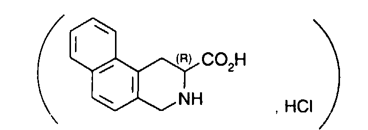

Le produit attendu est obtenu selon le procédé décrit dans la préparation A à partir de β-1-naphtyl-D-alanine en utilisant de l'acide chlorhydrique concentré au lieu de l'acide chlorhydrique 1N.
*Point de fusion : 174°*C

### Préparations F et G :

### Préparation F : Acide 1,2,3,4-tétrahydro-1-heptyl-β-carboline-(3R)-carboxylique, isomère α

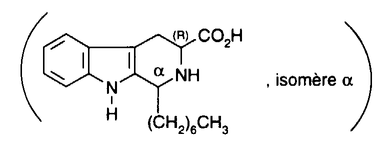

### Préparation G : Acide 1,2,3,4-tétrahydro-1-heptyl-β-carboline-(3R)-carboxylique, isomère β

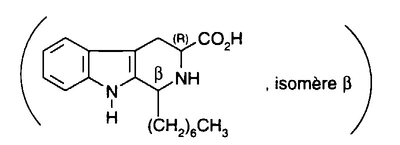

Un mélange contenant 147 mmoles de D-tryptophane, 294 ml d'acide sulfurique 1N, 300 ml d'éthanol et 294 mmoles d'octanal est agité à 45°C pendant 72 heures. La solution est concentrée et le résidu est repris par un mélange eau/méthanol/ammoniaque. Le méthanol est évaporé à 45°C et le précipité est filtré, lavé à l'eau et séché. Le solide obtenu est solubilisé à chaud dans du méthanol. Après refroidissement, le composé de la préparation F cristallise. II est alors filtré et séché.
*Préparation F: Point de fusion : 206°C*
Le jus de filtration est alors concentré à 45°C et le résidu obtenu est purifié par chromatographie sur gel de silice en utilisant comme éluant un mélange dichlorométhane/méthanol/acide acétique (90/10/1). On obtient alors le composé de la préparation G.
*Préparation G : Point de fusion : 170°C*

### Préparation H : Acide 1,2,3,4-tétrahydro-β-carboline-(3S)-carboxylique

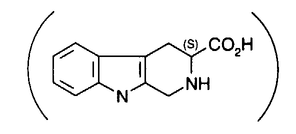

Le produit attendu est synthétisé selon le procédé décrit dans la préparation B à partir de (L)-tryptophane.

### Préparation I :Acide 1-méthyl-4,5,6,7-tétrahydroimidazo[4,5-c]pyridine-(6R)-carboxylique

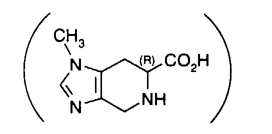

Le produit attendu est synthétisé selon le procédé décrit dans la préparation A à partir de (D)-(N^{π}-méthyl)histidine.

### Préparation J: Acide 3-méthyl-4,5,6,7-tétrahydroimidazo[4,5-c]pyridine-(6R)-carboxylique

Le produit attendu est obtenu selon le procédé décrit dans la préparation A à partir du (D)-(N^{τ}-méthyl)histidine.

### Préparation K : Acide 4,5,6,7-tétrahydrothiéno[2,3-c]pyridine-(5R)-carhoxylique, chlorhydrate

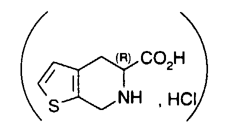

Le produit attendu est préparé selon le procédé décrit dans la préparation A à partir de β-3-thiénylalanine synthétisé selon la méthode décrite par M.S. Allen (Synth. Comm., 22 (14), 2077-2102, 1992).

### Préparation L : Acide 4,5,6,7-tétrahydrofuro[2,3-c]pyridine-(5R)-carboxylique, chlorhydrate

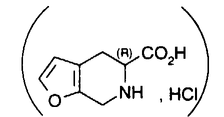

Le produit attendu est préparé selon le procédé décrit dans la préparation A à partir de β-3-furoalanine synthétisé selon la méthode décrite par M.S. Allen (Synth. Comm., 22 (14), 2077-2102, 1992).

### EXEMPLE 1 : 5-(4-Méthoxybenzènesulfonyl)-4,5,6,7-tétrahydrothiéno[3,2-c]pyridine-(6R)-(N-hydroxy)carboxamide

### Stade A : Acide 5-(4-méthoxybenzènesulfonyl)-4,5,6,7-tétrahydrothiéno[3,2-c]pyridine-(6R)-carboxylique

Un mélange contenant 36 mmoles du composé décrit dans la préparation A, 54 mmoles de triéthylamine, 43 mmoles de chlorure de l'acide 4-méthoxybenzène sulfonique dans 100 ml de dioxane et 80 ml d'eau, est agité une nuit à 20°C. Le mélange est alors versé dans 500 ml d'eau glacée et 500 ml d'acide chlorhydrique 4N. Après 15 minutes d'agitation, le précipité est filtré, rincé à l'eau, séché et conduit au produit attendu.
*Point de fusion : 110°C*

### Stade B : 5-(4-Méthoxybenzènesulfonyl)-4,5,6,7-tétrahydrothiéno[3,2-c]pyridine-(6R)-(N-allyloxy)carboxamide

10 mmoles du composé obtenu au stade précédent, 30 mmoles de chlorhydrate de 0-allylhydroxylamine, 17 ml de diisopropyléthylamine, 10 mmoles d'hydroxybenzotriazole et 12 mmoles de 0-(Benzotriazol-1-yl)-N,N,N',N'-tétraméthyluronium tétrafluoroborate (TBTU) sont agités 5 heures à 20°C. Le mélange est alors lavé à l'eau, à l'acide chlorhydrique puis par une solution aqueuse saturée d'hydrogénocarbonate de sodium. Après séchage puis évaporation, le résidu est repris puis purifié par chromatographie sur gel de silice en utilisant comme éluant un mélange dichlorométhane/acétate d'éthyle (95/5 puis 70/30). Après séchage et évaporation, on obtient le produit attendu.

### Stade C : 5-(4-Méthoxybenzènesulfonyl)-4,5,6,7-tétrahydrothiéno[3,2-c]pyridine-(6R)-(N-hydroxy)carboxamide

3,9 mmoles du composé obtenu au stade précédent, 140 mg de (Ph₃P)₂PdCl₂, 0,7 ml d'acide acétique dans 50 ml de dichlorométhane sont agités 10 minutes à 20°C. 2,2 ml d'hydrure de tributylétain sont alors ajoutés et l'ensemble est maintenu 15 minutes sous agitation à 20°C. Le milieu réactionnel est alors lavé à l'eau puis le solvant est évaporé. Le résidu est repris au dichlorométhane. La phase organique est lavée à l'acide chlorhydrique 1N puis par une solution saturée d'hydrogénocarbonate de sodium. Après séchage et évaporation, le résidu est purifié par chromatographie sur gel de silice en utilisant comme éluant un mélange dichlorométhane/méthanol (95/5). On obtient alors le produit attendu.
*Microanalyse élémentaire :*

| | *C %* | *H %* | *N %* | *S %* |
|---|---|---|---|---|
| *calculé* | *48,90* | *4,38* | *7,60* | *17,41* |
| *trouvé* | *49,84* | *4,49* | *7,23* | *16,64* |

*Spectre de masse : FAB [M+H]*^{*+*} *: m/z = 369*

### EXEMPLE 2 : 2-(4-Méthoxybenzênesulfonyl)-1,2,3,4-tétrahydro-β-carboline-(3R)-(N-hydroxy)carboxamide

### Stade A : Acide 2-(4-méthoxybenzènesulfonyl)-1,2,3,4-tétrahydro-β-carboline-(3R)-carboxylique

Le produit attendu est obtenu selon le procédé décrit au stade A de l'exemple 1 en utilisant comme produit de départ le composé décrit dans la préparation B.
*Point fe fusion : 154°C*

### Stade B : 2-(4-Méthoxybenzènesulfonyl)-1,2,3,4-tétrahydro-β-carboline-(3R)-(N-allyloxy)carboxamide

Le produit attendu est obtenu selon le procédé décrit au stade B de l'exemple 1 à partir du composé décrit au stade précédent.

### Stade C : 2-(4-Méthoxybenzènesulfonyl)-1,2,3,4-tétrahydro-β-carboline-(3R)-(N-hydroxy)carboxamide

Le produit attendu est obtenu selon le procédé décrit au stade C de l'exemple 1 en utilisant le composé décrit au stade précédent.
*Point de fusion : 122°C*
*Microanalyse élémentaire :*

| | *C %* | *H %* | *N %* | *S %* |
|---|---|---|---|---|
| *calculé* | *56,84* | *4,78* | *10,47* | *7,99* |
| *trouvé* | *56,40* | *4,56* | *10,34* | *8,25* |

### EXEMPLE 3 : 2-(4-Méthoxybenzènesulfonyl)-1,2,3,4-tétrahydrobenzo[b]thiéno [2,3-c]pyridine-(3R)-(N-hydroxy)carboxamide, sel de sodium

Le produit attendu est obtenu selon le procédé décrit dans l'exemple 1 en utilisant au stade A le produit décrit dans la préparation C.

### Stade A : Acide 2-(4-méthoxybenzènesulfonyl)-1,2,3,4-tétrahydrobenzo[b]thiéno[2,3-c]pyridine-(3R)carboxylique

### Stade B : 2-(4-Méthoxybenzènesulfonyl)-1,2,3,4-tétrahydrobenzo[b]thiéno-[2,3-c]pyridine-(3R)-(N-allyloxy)carboxamide

### Stade C : 2-(4-Méthoxybenzènesulfonyl)-1,2,3,4-tétrahydrobenzo[b]thiéno[2,3-c]pyridine-(3R)-(N-hydroxy)carboxamide, sel de sodium

*Point de fusion : ≈ 118°C*
*Spectre de masse : FAB [M+Na*^{*+*}*] = 441*

### EXEMPLE 4 : 2-(4-Méthoxybenzènesulfonyl)-1,2,3,4-tétrahydrobenzo[h]isoquinoléine-(3R)-(N-hydroxy)carboxamide

Le produit attendu est obtenu selon le procédé décrit dans l'exemple 1 en utilisant au stade A le produit décrit dans la préparation D.

### Stade A : Acide 2-(4-méthoxybenzènesulfonyl)-1,2,3,4-tétrahydrobenzo[h]isoquinoléine-(3R)-carboxylique

### Stade B : 2-(4-Méthoxybenzènesulfonyl)-1,2,3,4-tétrahydrobenzo[h]isoquinoléine-(3R)-(N-allyloxy)carboxamide

### Stade C : 2-(4-Méthoxybenzènesulfonyl)-1,2,3,4-tétrahydrobenzo[h]isoquinoléine-(3R)-(N-hydroxy)carboxamide

### Spectre de masse : FAB [M+H]⁺ : m/z = 413

### EXEMPLE 5 : 2-(4-Méthoxybenzènesulfonyl)-1,2,3,4-tétrahydrobenzo[f]isoquinoléine-(3R)-(N-hydroxy)carboxamide

### Stade A : Acide 2-(4-méthoxybenzènesulfonyl)-1,2,3,4-tétrahydrobenzo[f]isoquinoléine-(3R)-carboxylique

### Stade B : 2-(4-Méthoxybenzènesulfonyl)-1,2,3,4-tétrahydrobenzo[f] isoquinoléine-(3R)-(N-allyloxy)carboxamide

### Stade C : 2-(4-Méthoxybenzènesulfonyl)-1,2,3,4-tétrahydrobenzo[f]isoquinoléine-(3R)-(N-hydroxy)carboxamide

### Spectre de masse : FAB [M+H]⁺ : m/z = 413

### EXEMPLE 6 : 2-(4-Méthoxybenzènesulfonyl)-1,2,3,4-tétrahydro-1-heptyl-β-carboline-(3R)-(N-hydroxy)carboxamide, isomère α

Le produit attendu est obtenu selon le procédé décrit dans l'exemple 1 en utilisant au stade A le produit décrit dans la préparation F.

### Stade A : Acide 2-(4-méthoxybenzènesulfonyl)-1,2,3,4-tétrahydro-1-heptyl-β-carboline-(3R)-carboxylique, isomère α

### Stade B : 2-(4-Méthoxybenzènesulfonyl)-1,2,3,4-tétrahydro-1-heptyl-β-carboline-(3R)-(N-allyloxy)carboxamide, isomère α

### Stade C : 2-(4-Méthoxybenzènesulfonyl)-1,2,3,4-tétrahydro-1-heptyl-β-carboline-(3R)-(N-hydroxy)carboxamide, isomère α

### Spectre de masse : FAB [M+H]⁺ : m/z = 500

### EXEMPLE 7 : 2-(4-Méthoxybenzènesulfonyl)-1,2,3,4-tétrahydro-1-heptyl-β-carboline-(3R)-(N-hydroxy)carboxamide, isomère β

Le produit attendu est obtenu selon le procédé décrit dans l'exemple 1 en utilisant au stade A le produit décrit dans la préparation G.

### Stade A : Acide 2-(4-méthoxybenzènesulfonyl)-1,2,3,4-tétrahydro-1-heptyl-β-carboline-(3R)-carboxylique, isomère β

### Stade B : 2-(4-Méthoxybenzènesulfonyl)-1,2,3,4-tétrahydro-1-heptyl-β-carboline-(3R)-(N-allyloxy)carboxamide, isomère β

### Stade C : 2-(4-Méthoxybenzènesulfonyl)-1,2,3,4-tétrahydro-1-heptyl-β-carboline-(3R)-(N-hydroxy)carboxamide, isomère β

### Spectre de masse : FAB [M+H]⁺ : m/z = 500

### EXEMPLE 8 : 2-(4-Méthoxybenzènesuifonyl)-1,2,3,4-tétrahydro-β-carboline-(3S)-(N-hydroxy)carboxamide

Le produit attendu est obtenu selon le procédé décrit dans l'exemple 1 en utilisant au stade A le produit décrit dans la préparation H.

### Stade A : Acide 2-(4-méthoxybenzènesulfonyl)-1,2,3,4-tétrahydro-β-carboline-(3S)-carboxylique

### Point de fusion : 230°C

### Stade B : 2-(4-Méthoxybenzènesulfonyl)-1,2,3,4-tétrahydro-β-carboline-(3S)-(N-benzyloxy)carboxamide

A ce stade, l'O-allylhydroxylamine a été remplacé par l'O-benzylhydroxylamine.

### Stade C : 2-(4-Méthoxybenzènesulfonyl)-1,2,3,4-tétrahydro-β-carboline-(3S)-(N-hydroxy)carboxamide

### Spectre de masse : FAB [M+H]⁺ : m/z = 402

### EXEMPLE 9 : 1-(4-Méthoxyphénylsulfonyl)indoline-(2R)-(N-hydroxy)carboxamide

Le produit attendu est obtenu selon le procédé décrit dans l'exemple 1 en utilisant au stade A l'acide indoline-(2R)-carboxylique.

### Stade A : Acide 1-(4-méthoxyphénylsulfonyl)indoline-(2R)-carboxylique

### Stade B : 1-(4-Méthoxyphénylsulfonyl)indoline-(2R)-(N-benzyloxy)carboxamide

A ce stade, l'O-allylhydroxylamine a été remplacé par l'O-benzylhydroxylamine.

### Stade C : 1-(4-Méthoxyphénylsulfonyl)indoline-(2R)-(N-hydroxy)carboxamide

A ce stade, la déprotection est réalisée dans du méthanol, à pression atmosphérique en utilisant Pd(OH)₂ comme catalyseur.
*Microanalyse élémentaire :*

| | *C %* | *H %* | *N %* | *S %* |
|---|---|---|---|---|
| *calculé* | *55,16* | *4,63* | *8,04* | *9,20* |
| *trouvé* | *55,25* | *4,87* | *7,85* | *8,52* |

### EXEMPLE 10 : 2-(4-Méthoxybenzoyl)-1,2,3,4-tétrahydroisoquinoléine-(3R)-(N-hydroxy)carboxamide

### Stade A : Acide 2-(4-méthoxybenzoyl)-1,2,3,4-tétrahydroisoquinoléine-(3R)-carboxylique

Le produit attendu est obtenu selon le procédé décrit au stade A de l'exemple 1 à partir d'acide 1,2,3,4-tétrahydroisoquinoléine-(3R)-carboxylique et du chlorure de l'acide (4-méthoxy)benzoïque.
*Point de fusion : 198°C*

### Stade B : 2-(4-Méthoxybenzoyl)-1,2,3,4-tétrahydroisoquinoléine-(3R)-(N-benzyloxy)carboxamide

Le produit attendu est obtenu selon le procédé décrit au stade B de l'exemple 1 à partir du composé décrit au stade précédent et de l'O-benzylhydroxylamine.

### Stade C : 2-(4-Méthoxybenzoyl)-1,2,3,4-tétrahydroisoquinoléine-(3R)-(N-hydroxy)carboxamide

Le produit attendu est obtenu selon le procédé décrit au stade C de l'exemple 1 à partir du composé décrit au stade précédent.
*Microanalyse élémentaire :*

| | *C %* | *H %* | *N %* |
|---|---|---|---|
| *calculé* | *66,25* | *5,56* | *8,75* |
| *trouvé* | *66,10* | *5,64* | *8,58* |

### EXEMPLE 11 : 1-Méthyl-5-(4-méthoxybenzènesulfonyl)-4,5,6,7-tétrahydroimidazo[4,5-c]pyridine-(6R)-(N-hydroxy)carboxamide, chlorhydrate

Le produit attendu est obtenu selon le procédé décrit dans l'exemple 1 en utilisant au stade A le produit décrit dans la préparation 1.

### Stade A : Acide 1-méthyl-5-(4-méthoxybenzènesulfonyl)-4,5,6,7-tétrahydroimidazo[4,5-c]pyridine-(6R)-carboxylique

### Stade B : 1-Méthyl-5-(4-méthoxybenzènesulfonyl)-4,5,6,7-tétrahydroimidazo[4,5-c]pyridine-(6R)-(N-allyloxy)carboxamide

### Stade C : 1-Méthyl-5-(4-méthoxybenzènesulfonyl)-4,5,6,7-tétrahydroimidazo[4,5-c]pyridine-(6R)-(N-hydroxy)carboxamide, chlorhydrate

*Microanalyse élémentaire :*

| | *C %* | *H %* | *N %* | *Cl %* | *S %* |
|---|---|---|---|---|---|
| *calculé* | *44,72* | *4,75* | *13,91* | *8,80* | *7,96* |
| *trouvé* | *44,78* | *4,69* | *13,72* | *8,84* | *7,95* |

### EXEMPLE 12 : 3-Méthyl-5-(4-méthoxybenzènesulfonyl)-4,5,6,7-tétrahydroimidazo[4,5-c]pyridine-(6R)-(N-hydroxy)carboxamide, chlorhydrate

Le produit attendu est obtenu selon le procédé décrit dans l'exemple 1 en utilisant au stade A le produit décrit dans la préparation J.

### Stade A : Acide 3-méthyl-5-(4-méthoxybenzènesulfonyl)-4,5,6,7-tétrahydroimidazo[4,5-c]pyridine-(6R)-carboxylique

### Stade B : 3-Méthyl-5-(4-méthoxybenzènesulfonyl)-4,5,6,7-tétrahydroimidazo[4,5-c]pyridine-(6R)-(N-allyloxy)carboxamide

### Stade C : 3-Méthyl-5-(4-méthoxybenzènesulfonyl)-4,5,6,7-tétrahydroimidazo[4,5-c]pyridine-(6R)-(N-hydroxy)carboxamide, chlorhydrate

*Microanalyse élémentaire :*

| | *C %* | *H %* | *N %* | *Cl %* | *S %* |
|---|---|---|---|---|---|
| *calculé* | *44,72* | *4,75* | *13,91* | *8,80* | *7,96* |
| *trouvé* | *44,77* | *4,70* | *13,80* | *9,11* | *8,10* |

### EXEMPLE 13 : 2-[(3-Phényl)propylsulfonyl]-1,2,3,4-tétrahydroisoquinoléine-(3R)-(N-hydroxy)carboxamide

Le produit attendu est obtenu selon le procédé décrit dans l'exemple 10 en utilisant au stade A le chlorure de l'acide 3-phénylpropylsulfonique.

### Stade A : Acide 2-[(3-phényl)propylsulfonyl]-1,2,3,4-tétrahydroisoquinoléine-(3R)-carboxylique

### Stade B : 2-[(3-Phényl)propulsulfonyl]-1,2,3,4-tétrahydroisoquinoléine-(3R)-(N-benzyloxy)carboxamide

### Stade C : 2-[(3-Phényl)propylsulfonyl]-1,2,3,4-tétrahydroisoquinoléine-(3R)-(N-hydroxy)carboxamide

*Microanalyse élémentaire :*

| | *C %* | *H %* | *N %* | *S %* |
|---|---|---|---|---|
| *calculé* | *60,94* | *5,92* | *7,48* | *8,56* |
| *trouvé* | *60,67* | *5,91* | *7,58* | *8,36* |

### EXEMPLE 14 : 2-[(4-Méthoxybenzène)aminosulfonyl]-1,2,3,4- tétrahydroisoquinoléine-(3R)-(N-hydroxy)carboxamide

Le produit attendu est obtenu selon le procédé décrit dans l'exemple 10 en utilisant au stade A le chlorure de l'acide 4-méthoxybenzèneaminosulfonique.

### Stade A : Acide 2-[(4-méthoxybenzène)aminosulfonyl]-1,2,3,4-tétrahydroisoquinoléine-(3R)-carboxylique

### Stade B : 2-[(4-Méthoxybenzène)aminosulfonyl]-1,2,3,4-tétrahydroisoquinoléine-(3R)-(N-benzyloxy)carboxamide

### Stade C : 2-[(4-Méthoxybenzène)aminosulfonyl]- 1,2,3,4-tétrahydroisoquinoléine-(3R)-(N-hydroxy)carboxamide

*Microanalyse élémentaire :*

| | *C %* | *H %* | *N %* | *S %* |
|---|---|---|---|---|
| *calculé* | *54,10* | *5,07* | *11,13* | *8,50* |
| *trouvé* | *54,13* | *5,15* | *10,92* | *8,16* |

### EXEMPLE 15 : 6-(4-Méthoxybenzènesulfonyl)-4,5,6,7-tétrakydrothiéno[2,3-c]pyridine-(5R)-(N-hydroxy)carboxamide

Le produit attendu est obtenu selon le procédé décrit dans l'exemple 1 à partir du composé décrit dans la préparation K.
*Microanalyse élémentaire :*

| | *C %* | *H %* | *N %* | *S %* |
|---|---|---|---|---|
| *calculé* | *48,90* | *4,38* | *7,60* | *17,41* |
| *trouvé* | *49,08* | *4,66* | *7,43* | *17,19* |

### EXEMPLE 16 : 6-(4-Trifluorométhoxybenzènesulfonyl)-4,5,6,7-tétrahydrothiéno[2,3-c]pyridine-(5R)-(N-hydroxy)carboxamide

Le produit attendu est obtenu selon le procédé décrit dans l'exemple 1, à partir du composé décrit dans la préparation K et du chlorure de l'acide 4-trifluorométhoxybenzènesulfonique.
*Point de fusion : 136-138°C*
*Microanalyse élémentaire :*

| | *C %* | *H %* | *N %* | *S %* |
|---|---|---|---|---|
| *calculé* | *42,65* | *3,10* | *6,63* | *15,18* |
| *trouvé* | *42,63* | *3,30* | *6,51* | *15,04* |

### EXEMPLE 17 : 5-(4-Phénylbenzènesulfonyl)-4,5,6,7-tétrahydrothiéno[3,2-c]pyridine-(6R)-(N-hydroxy)carboxamide

Le produit attendu est obtenu selon le procédé décrit dans l'exemple 1 à partir du composé décrit dans la préparation A et du chlorure de l'acide 4-phénylbenzènesulfonique.
*Microanalyse élémentaire :*

| | *C %* | *H %* | *N %* | *S %* |
|---|---|---|---|---|
| *calculé* | *57,95* | *4,38* | *6,76* | *15,47* |
| *trouvé* | *57,61* | *4,77* | *6,42* | *15,22* |

### EXEMPLE 18 : 5-(4-Trifluorométhoxybenzènesulfonyl)-4,5,6,7-tétrahydrothiéno[3,2-c]pyridine-(6R)-(N-hydroxy)carboxamide

Le produit attendu est obtenu selon le procédé décrit dans l'exemple 1 à partir du composé décrit dans la préparation A et du chlorure de l'acide 4-trifluorométhoxybenzènesulfonique.
*Spectre de masse : FAB*^{*+*} *: [M+H]*^{*+*} *: m/z = 423*

### EXEMPLE 19 : 5-[(Napht-2-yl)sulfonyl]-4,5,6,7-tétrahydrothiéno[3,2-c]pyridine-(6R)-(N-hydroxy)carboxamide

Le produit attendu est obtenu selon le procédé décrit dans l'exemple 1 à partir du composé décrit dans la préparation A et du chlorure de l'acide 2-naphtalènesulfonique.
*Spectre de masse : FAB*^{*+*} *: [M+H]+: m/z = 389*

### EXEMPLE 20 : 2-(4-Méthoxybenzènesulfonyl)-1,2,3,4-tétrahydrobenzo[b]thiénof[2,3-c]pyridine-(3R)-(N-méthoxy)carboxamide

Le produit attendu est obtenu en faisant réagir le composé décrit au stade A de l'exemple 3 avec la N-méthoxyamine en présence du réactif du couplage DCC-HOBT.
*Point de fusion : 202°C*
*Microanalyse élémentaire :*

| | *C %* | *H %* | *N %* | *S %* |
|---|---|---|---|---|
| *calculé* | *55,52* | *4,66* | *6,48* | *14,83* |
| *trouvé* | *55,89* | *4,78* | *6,47* | *15,05* |

### EXEMPLE 21 : 2-(4-Méthoxybenzènesulfonyl)-1,2,3,4-tétrahydrobenzo[b]thiéno[2,3-c]pyridine-(3R)-(N-hydroxy-N-méthyl)carboxamide

Le produit attendu est obtenu en faisant réagir le composé décrit au stade A de l'exemple 3 avec la (N-méthyl)hydroxylamine en présence du réactif de couplage DCC-HOBT.
*Point de fusion : 224°C*
*Microanalyse élémentaire :*

| | *C %* | *H %* | *N %* | *S %* |
|---|---|---|---|---|
| *calculé* | *55,54* | *4,66* | *6,48* | *14,83* |
| *trouvé* | *55,86* | *4,78* | *6,39* | *14,60* |

### EXEMPLE 22 : 2-(4-Méthoxybenzènesulfonyl)-1,2,3,4-tétrahydrobenzo[b]thiéno[2,3-c]pyridine-(3R)-(N-méthoxy-N-méthyl)carboxamide

Le produit attendu est obtenu en faisant réagir le composé décrit au stade A de l'exemple 3 avec la N-méthoxy-N-méthylamine en présence du réactif de couplage HOBT-TBTU.
*Microanalyse élémentaire :*

| | *C %* | *H %* | *N %* | *S %* |
|---|---|---|---|---|
| *calculé* | *56,49* | *4,97* | *6,27* | *14,36* |
| *trouvé* | *56,47* | *5,09* | *6,32* | *14,06* |

### EXEMPLE 23 : 5-(4-Méthoxybenzènesulfonyl)-4,5,6,7-tétrahydroimidazo[4,5-c]pyridine(6R)-(N-hydroxy)carboxamide, chlorhydrate

Le produit attendu est obtenu selon le procédé décrit dans l'exemple 1 à partir de la Spinacine et transformé en chlorhydrate correspondant.
*Microanalyse élémentaire :*

| | *C %* | *H %* | *N %* | *Cl %* | *S %* |
|---|---|---|---|---|---|
| *calculé* | *43,25* | *4,41* | *14,41* | *9,12* | *8,25* |
| *trouvé* | *42,91* | *4,52* | *13,85* | *9,55* | *8,02* |

### EXEMPLE 24 : 2-(4-Méthoxybenzènesulfonyl)-9-méthyl-1,2,3,4.tétrahydro-β-carboline-(3R)-(N-hydroxy)carboxamide

Le produit attendu est obtenu selon le procédé décrit dans l'exemple 1 à partir de l'acide 9-méthyl- 1,2,3,4-tétrahydro-β-carboline-(3R)-carboxylique.
*Point de fusion : 214°C*
*Microanalyse élémentaire :*

| | *C %* | *H %* | *N %* | *S %* |
|---|---|---|---|---|
| *calculé* | *57,82* | *5,09* | *10,11* | *7,72* |
| *trouvé* | *57,35* | *5,28* | *9,57* | *7,74* |

### EXEMPLE 25 : 2-(4-Méthoxybenzènesulfonyl)-9-benzyl-1,2,3,4-tétrahydro-β-carboline-(3R)-(N-hydroxy)carboxamide

Le produit attendu est obtenu selon le procédé décrit dans l'exemple 1 à partir de l'acide 9-benzyl-1,2,3,4-tétrahydro-β-carboline-(3R)-carboxylique.
*Microanalyse élémentaire :*

| | *C %* | *H %* | *N %* | *S %* |
|---|---|---|---|---|
| *calculé* | *63,53* | *5,13* | *8,55* | *6,52* |
| *trouvé* | *63,24* | *5,12* | *8,34* | *6,13* |

### EXEMPLE 26 : 2-(4-Phénylbenzènesulfonyl)-9-benzyl-1,2,3,4-tétrahydro-β-carboline-(3R)-(N-hydroxy)carboxamide

Le produit attendu est obtenu selon le procédé décrit dans l'exemple 1 à partir de l'acide 9-benzyl-1,2,3,4-tétrahydro-β-carboline-(3R)-carboxylique et du chlorure de l'acide 4-phénylbenzènesulfonique.
*Spectre de masse : FAB*^{*+*} *: [M+H]*^{*+*} *: m/z* = *538*

### EXEMPLE 27 : 2-(4-Pentylbenzènesulfonyl)-9-benzyl-1,2,3,4-tétrahydro-β-carboline-(3R)-(N-hydroxy)carboxamide

Le produit attendu est obtenu selon le procédé décrit dans l'exemple 1 à partir de l'acide 9-benzyl- 1,2,3,4-tétrahydro-β-carboline-(3R)-carboxylique et du chlorure de l'acide 4-pentylbenzènesulfonique.
*Point de fusion : 94°C*
*Microanalyse élémentaire :*

| | *C %* | *H %* | *N %* | *S %* |
|---|---|---|---|---|
| *calculé* | *67,77* | *6,26* | *7,90* | *6,03* |
| *trouvé* | *67,96* | *6,29* | *7,62* | *5,92* |

### EXEMPLE 28 : 6-(4-Méthoxybenzènesulfonyl)-4,5,6,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.
*Point de fusion : 149°C*
*Microanalyse élémentaire :*

| | *C %* | *H %* | *N %* | *S %* |
|---|---|---|---|---|
| *calculé* | *51,13* | *4,58* | *7,95* | *9,10* |
| *trouvé* | *51,18* | *4,60* | *7,77* | *9,11* |

### EXEMPLE 29 : 2-(4-Méthoxybenzènesulfonyl)-1,2,3,4-tétrahydroisoquinoléine-(3R)-carbohydroximamide

### Stade A : Acide 2-(4-méthoxybenzènesulfonyl-1,2,3,4-tétrahydroisoquinoléine-(3R)-carboxylique

Le produit attendu est obtenu selon le procédé décrit au stade A de l'exemple 1 à partir de l'acide 1,2,3,4-tétrahydroisoquinoléine-(3R)-carboxylique et du chlorure de l'acide 4-méthoxybenzènesulfonique.

### Stade B : 2-(4-Méthoxybenzènesulfonyl)-1,2,3,4-tétrahydroisoquinoléine-(3R)-carboxamide

Le produit attendu est obtenu en faisant réagir le composé décrit au stade précédent avec du chloroformiate d'éthyle en milieu tétrahydrofurane/triéthylamine puis avec de l'ammoniaque.

### Stade C : (3R)-2-(4-Méthoxybenzènesulfonyl)3-cyano-1,2,3,4-tétrahydroisoquinoléine

Le produit attendu est obtenu en faisant réagir le composé décrit au stade précédent dans de la pyridine en présence de POCl₃.

### Stade D : 2-(4-Méthoxybenzènesulfonyl)-1,2,3,4-tétrahydroisoquinoléine-(3R)-carbohydroximamide

Le produit attendu est obtenu en faisant réagir le composé décrit au stade précédent avec de l'hydroxylamine.
*Microanalyse élémentaire :*

| | *C %* | *H %* | *N %* | *S %* |
|---|---|---|---|---|
| *calculé* | *56,50* | *5,30* | *11,63* | *8,87* |
| *trouvé* | *56,57* | *5,38* | *11,03* | *8,95* |

### EXEMPLE 30 : 2-(4-Trifluorométhoxybenzènesulfonyl)-4,5,6,7-tétrahydrothiéno[3,2-c]pyridine-(6R)-(N-hydroxy-N-méthyl)carboxamide

Le produit attendu est obtenu selon le procédé décrit dans l'exemple 21 à partir du composé décrit au stade A de l'exemple 1.
*Microanalyse élémentaire :*

| | *C %* | *H %* | *N %* | *S %* |
|---|---|---|---|---|
| *calculé* | *44,03* | *3,46* | *6,42* | *14,69* |
| *trouvé* | *44,02* | *3,13* | *6,39* | *14,80* |

### EXEMPLE 31 : 5-(4-Méthoxybenzènesulfonyl)-7-hydroxy-4,5,6,7-tétrahydrothiéno[3,2-c]pyridine-(6R)-(N-hydroxy)carboxamide

Le produit attendu est obtenu selon le procédé décrit dans l'exemple 1 à partir d'acide 7-hydroxy-4,5,6,7-tétrahydrothiéno [3,2-c]pyridine-(6R)-carboxylique.

### EXEMPLE 32 : 1-Méthyl-5-(4-méthoxybenzènesulfonyl)-4,5,6,7-tétrahydropyrrolo[3,2-c]pyridine-(6R)-(N-hydroxy)carboxamide

Le produit attendu est obtenu selon le procédé décrit dans l'exemple 1 à partir de l'acide 1-méthyl-4,5,6,7-tétrahydropyrrolo[3,2-c]pyridine-(6R)-carboxylique.

### EXEMPLE 33 : 6-(4-Méthoxybenzènesulfonyl)-4,5,6,7-tétrahydropyrrolo[2,3-c]pyridine-(5R)-(N-hydroxy)carboxamide

Le produit attendu est obtenu selon le procédé décrit dans l'exemple 1 à partir de l'acide 4,5,6,7-tétrahydropyrrolo[2,3-c]-pyridine(5R)-carboxylique.

### EXEMPLE 34 : 6-(4-Méthoxybenzènesulfonyl)-5,6,7,8-tétrahydropyrido[3,2-c]pyridine-(7R)-(N-hydroxy)carboxamide

Le produit attendu est obtenu selon le procédé décrit dans l'exemple 1 à partir de l'acide 5,6,7,8-tétrahydropyrido[3,2-c]pyridine-(7R)-carboxylique.

### EXEMPLE 35 : 7-(4-Méthoxybenzènesulfonyl)-5,6,7,8-tétrahydropyrido[2,3-c]pyridine-(6R)-(N-hydroxy)carboxamide

Le produit attendu est obtenu selon le procédé décrit dans l'exemple 1 à partir de l'acide 5,6,7,8-tétrahydropyrido[2,3-c]pyridine-(7R)-carboxylique.

### EXEMPLE 36 : 5-(4-Trifluorométhoxyphénylsulfonyl)-4,5,6,7-tétrahydroimidazo[4,5-c]pyridine-(6R)-(N-hydroxy)carboxamide

Le produit attendu est obtenu selon le procédé décrit dans l'exemple 1 à partir de Spinacine et du chlorure de l'acide 4-trifluorométhoxyphénylsulfonique.

### EXEMPLE 37 : 5-(4-Phénylbenzènesulfonyl)-4,5,6,7-tétrahydroimidazo[4,5-c]pyridine-(6R)-(N-hydroxy)carboxamide

Le produit attendu est obtenu selon le procédé décrit dans l'exemple 1 à partir de Spinacine et du chlorure de l'acide 4-phénylbenzènesulfonique.

### EXEMPLE 38: 6-(4-Trifluorométhoxybentènesulfonyl)-4,5,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 39 : 6-(4-Phénylbenzènesulfonyl)-4,5,6,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 40: 6-(4-Pyridylsulfonyl)-4,5,6,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 41 : 6-(3-Pyridylsulfonyl)-4,5,6,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 42 : 6-[4-(Pyridin-4-yl)phénylsulfonyl]-4,5,6,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 43: 6-{4-[2-(Diméthylamino)éthoxy]phénylsulfonyl)-4,5,6,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 44: 6-{4(Pyrimidin-2-yl)phénylsulfonyl]-4,5,6,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 45: 6-{4-(Pyrimidin-5-yl)phénylsulfonyl]-4,5,7-tétrahydrofuro[2,3-c]pyridine-(5R)-(N-hydroxy)carboxamide

Le produit attendu est obtenu selon le procédé décrit dans l'exemple 1 à partir du composé décrit dans la préparation L.

### EXEMPLE 46 : 5-[4-(4-Fluorophényl)benzènesulfonyl]-4,5,6,7-tétrahydroimidazo[4,5-c]pyridine-(6R)-(N-hydroxy)carboxamide

Le produit attendu est obtenu selon le procédé décrit dans l'exemple 1 à partir de la Spinacine.

### EXEMPLE 47: 5-[4-(Pyridin-4-yl)phénylsulfonyl]-4,5,6,7-tétrahydroimidazo[4,5-c]pyridine-(6R)-(N-hydroxy)carboxamide

Le produit attendu est obtenu selon le procédé décrit dans l'exemple 1 à partir de la Spinacine.

### EXEMPLE 48 : 5-(4-Pyridylsulfonyl)-4,5,6,7-tétrahydroimidazo[4,5-c]pyridine-(6R)-(N-hydroxy)carboxamide

Le produit attendu est obtenu selon le procédé décrit dans l'exemple 1 à partir de la Spinacine.

### EXEMPLE 49 : 5-(3-Pyridylsulfonyl)-4,5,6,7-tétrahydroimidazo[4,5-c]pyridine-(6R)-(N-hydroxy)carboxamide

Le produit attendu est obtenu selon le procédé décrit dans l'exemple 1 à partir de la Spinacine.

### Etude pharmacologique des dérivés de l'invention

### EXEMPLE 50: Inhibition enzymatique des métalloprotéases

Les quatre enzymes humaines recombinantes MMP-1 (collagénase interstitielle), MMP-2 (gélatinase A de 72kda), MMP-3 (stromélysine 1) et MMP-9 (gélatinase B de 92kda) sont activées à l'AMPA (4-aminophénylmercuric acétate).
Les tests enzymatiques sont effectués avec un substrat peptido-mimétique : DnpProChaGlyCys(Me)HisAlaLys(Nma)NH2, clivé entre la Glycine et la Cystéine pour produire un dérivé fluorescent décrit par D.M. BICKETT et coll. (Anal. Biochem., 212, 58-64, 1993).
Les réactions conduites dans un tampon 50 mM Tris, 200 mM NaCl, 5 mM CaCl₂, 0,1 % Brij 35 à pH 7.7 sont initiées avec 20 µM de substrat dans un volume total de 100 µl à 37°C.
La fluorescence obtenue après six heures est lue en plaque 96-puits dans un fluorimètre équipé avec une combinaison de filtres de 340 nm et 440 nm pour l'excitation et l'émission.
Lors de ce test, la plupart des composés de l'invention ont présenté des IC₅₀ comprises entre 10 et 500 nM pour l'enzyme MMP-1, entre 0,01 et 50 nM pour les enzymes MMP-2, MMP-3 et MMP-9.

### EXEMPLE 51: Dégradation de la matrice cartilagineuse in vivo

Les composés de l'invention ont été étudiés sur un modèle de destruction de la matrice cartilagineuse induite par l'IL-1β. Les essais réalisés sur cartilage de cobaye concernent :
- d'une part la dégradation du collagène : dosage colorimétrique selon la technique de Grant (GRANT R.A. Estimation of OH-proline by the autoanalyser, J. Clin. Path. 1964, 17, 685) de la fraction d'OH-proline relarguée par le tissu mis en contact pendant 3 jours avec l'IL-1β (10 mg/ml) et du plasminogène (30 µg/ml),
- d'autre part la dégradation des protéoglycanes : mesure radio-isotopique de la fraction de glycosaminoglycanes relargés par le tissu au cours des 3 jours de contact avec l'IL-1β (10 mg/ml), le cartilage ayant été préalablement marqué au ^{3S}SO₄.

Les composés de l'invention ont été étudiés par adjonction au milieu de cultures pendant les 3 jours de l'essai. Pour des concentrations comprises entre 10-7 et 10-4M, ils se sont fortement opposés à la dégradation du collagène et des protéoglycanes. A titre d'exemple, les activités excercées par certains composés de l'invention sont les suivantes :

| | collagène | protéoglycanes |
|---|---|---|
| | % protection à 10⁻⁶ M | % protection à 3.10⁻⁵ M |
| exemple 1 | 98 % | 45 % |
| exemple 7 | 47 % | 100 % |
| exemple 15 | 98 % | 60 % |
| exemple 23 | 88 % | 24 % |
| exemple 28 | 79 % | 34 % |

### EXEMPLE 52: Angiogenèse in vitro

Des tronçons d'aorte thoracique de rats mâles Fischer 344 âgés de 8 à 12 semaines sont immergés dans un gel de collagène de type I selon la méthode de Nicosia et Ottinetti (1990). Après cinq jours de culture en milieu sans sérum, les préparations sont examinées au microscope et la formation de pseudo-vaisseaux quantifiée en terme de densité vasculaire après digitalisation et analyse d'image.
A titre d'exemple, lors de ce test, à 100 nM, le composé de l'exemple 1 a conduit à 87 % d'inhibition et le composé de l'exemple 28 à 85 % d'inhibition.

### EXEMPLE 53: Invasion in vitro

Les tests d'invasion sont réalisés selon la procédure suivante : des cellules murines de carcinome de Lewis (LLC) sont déposées sur la face supérieure d'un filtre Transwell recouvert d'une matrice extracellulaire artificielle et cultivées en milieu avec sérum pendant 24 h. Les cellules sont ensuite mises en présence du colorant vital 3-[4,5-diméthylthiazol-2-yl]-2,5-diphényltétrazolium bromide et éliminées de la face supérieure du filtre. Les cristaux de Formazan présents sous le filtre sont solubilisés dans un mélange concentré de sodium dodécyl sulphate et de diméthylformamide. L'absorption à 540 mm du mélange coloré correspondant est alors utilisée pour quantifier indirectement l'invasion cellulaire.

A titre d'exemple, lors de ce test, à 1 µM, le composé de l'exemple 1 a conduit à 45 % d'inhibition et le composé de l'exemple 28 à 44 % d'inhibition.

### EXEMPLE 54: Composition pharmaceutique

Formule de préparation pour 1000 comprimés dosés à 10 mg :

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 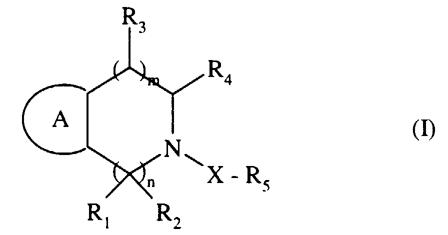 dans laquelle :
m, n, identiques ou différents, représentent 0, 1 ou 2,
R₁, R₂, identiques ou différents, représentent un atome d'hydrogène, un groupement alkyle (C₁-C₆) linéaire ou ramifié (éventuellement substitué par un groupement aryle), un groupement aryle, ou forment avec l'atome de carbone qui les porte un groupement carbonyle ou un groupement cycloalkyle (C₃-C₇),
R₃ représente un atome d'hydrogène, un groupement alkyle (C₁-C₆) linéaire ou ramifié, hydroxy, alkoxy (C₁-C₆) linéaire ou ramifié ou un groupement aryle,
R₄ représente l'un quelconque des groupements suivants :
■ -CO-NR₆-OR'₆
■ -CS-NR₆-OR'₆
■ 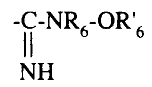 dans lesquels :
R₆ et R'₆, identiques ou différents, représentent un atome d'hydrogène ou un groupement alkyle (C₁-C₆) linéaire ou ramifié,
■ -CO₂R₇
■ -NH-CO-NH-OH
■ -NH-CH₂-CO₂R₇
■ 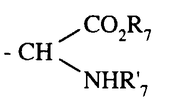
■ 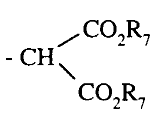 dans lesquels :
R₇, R'₇, identiques ou différents, représentent un atome d'hydrogène ou un groupement alkyle (C₁-C₆) linéaire ou ramifié (éventuellement substitué par un groupement aryle),
X représente -SO₂-, -CO- ou -SO₂NH-,
R₅ représente :
- un groupement alkyle (C₁-C₆) linéaire ou ramifié, éventuellement substitué par un ou plusieurs atomes d'halogène ou groupements hydroxy, alkoxy (C₁-C₆) linéaire ou ramifié, aryle ou -CO₂R₇ (dans lequel R₇ a la même signification que précédemment),
- un groupement cycloalkyle (C₃-C₇),
- un groupement aryle,
- ou, un groupement hétérocyclique,
A représente un cycle aryle (à la condition que ce cycle aryle soit différent d'un cycle phényle lorsque X représente SO₂, m et n représentent simultanément le nombre 1, R₄ représene -CO-NHOH, et R₅ représente un groupement aryle ou un groupement hétérocyclique), ou un hétérocycle,
leurs isomères ainsi que leurs sels d'addition à un acide ou à une base pharmaceutiquement acceptable.

2. Composés de formule (I) selon la revendication 1 tel que R₄ représente le groupement -CO-NR₆-OR'₆.

3. Composés de formule (I) selon la revendication 2 tel que R4 représente le groupement -CO-NHOH.

4. Composés de formule (I) selon la revendication 1 tels que A représente un cycle aryle choisi parmi phényle ou naphtyle, substitués ou non.

5. Composés de formule (I) selon la revendication 1 tels que A représente un hétérocycle choisi parmi les cycles thiophène, indole, furane, benzo[b]thiophène, imidazole, pyridine, benzofurane, pyrrole, quinoléine, substitués ou non.

6. Composés de formule (I) selon la revendication 1 tels que X représente -SO₂-.

7. Composés de formule (I) selon la revendication 1 tels que R5 représente un groupement aryle éventuellement substitué.

8. Composés de formule (I) selon la revendication 1 tels que m et n sont égaux à 1.

9. Composé de formule (I) selon la revendication 1 qui est le 5-(4-méthoxybenzènesulfonyl)-4,5,6,7-tétrahydrothiéno[3,2-c] pyridine-(6R)-(N-hydroxy)carboxamide.

10. Composé de formule (I) selon la revendication 1 qui est le 6-(4-méthoxybenzènesulfonyl)-4,5,6,7-tétrahydrothiéno[2,3-c]pyridine-(5R)-(N-hydroxy) carboxamide.

11. Composé de formule (I) selon la revendication 1 qui est le 5-(4-méthoxybenzènesulfonyl)-4,5,6,7-tétrahydroimidazo[4,5-c]pyridine-(6R)-(N-hydroxy) carboxamide.

12. Composé de formule (I) selon la revendication 1 qui est le 6-(4-méthoxybenzènesulfonyl)-4,5,6,7-tétrahydrofuro[2,3-c]pyridine-(5R)-(N-hydroxy) carboxamide.

13. Procédé de préparation des composés de formule (I) selon la revendication 1, caractérisé en ce que l'on utilise comme produit de départ un acide de formule (II), sous forme racémique ou d'isomère défini : 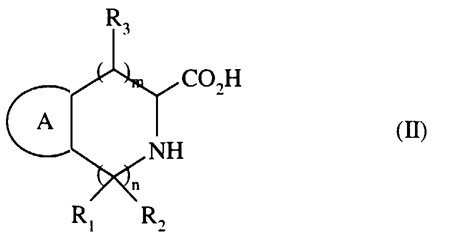 dans laquelle R₁, R₂, R₃, m, n et A ont la même signification que dans la formule (I), dont on substitue la fonction amine par un dérivé halogéné de formule (III) :
R₅ - X- Hal (III)
dans laquelle X et R₅ ont la même signification que dans la formule (I) et Hal représente un atome d'halogène,
pour conduire au composé de formule (I/a), cas particulier des composés de formule (I) : 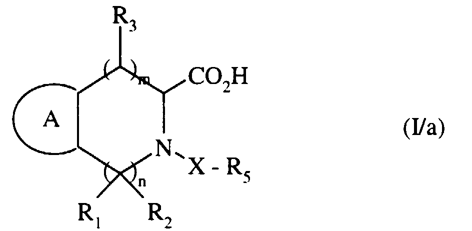 dans laquelle A, R₁, R₂, R₃, R₅, X, m et n ont la même signification que dans la formule (I),
composé de formule (I/a):
• (a) dont on transforme, si on le souhaite, la fonction acide en fonction ester correspondante,
• (b) ou, que l'on met en réaction avec une hydroxylamine-O- substituée
pour conduire, après déprotection de la fonction hydroxylamine et substitution éventuelle de la fonction hydroxylamine, au composé de formule (I/b), cas particulier des composés de formule (I) : 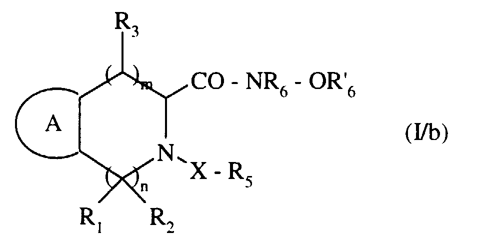 dans laquelle A, R₁, R₂, R₃, R₅, R₆, R'₆, X, m et n ont la même signification que dans la formule (I),
- composé de formule (I/b) que l'on soumet, si on le désire, à l'action du réactif de Lawesson dans l'orthoxylène,
pour conduire au composé de formule (I/c), cas particulier des composés de formule (I) : 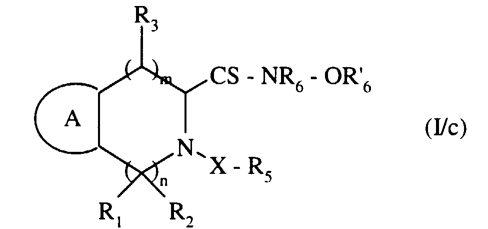 dans laquelle A, R₁, R₂, R₃, R₅, R₆, R'₆, X, m et n ont la même signification que dans la formule (I),
- composé de formule (I/c), que l'on soumet, si on le souhaite, à l'action de l'ammoniaque,
pour conduire au composé de formule (I/d), cas particulier des composés de formule (I) : 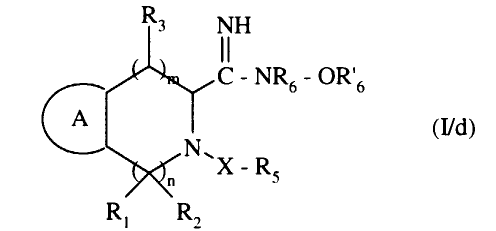 dans laquelle A, R₁, R₂, R₃, R₅, R₆, R'₆, X, m et n ont la même signification que dans la formule (I),
• (c) que l'on transforme en amine primaire de formule (IV) : 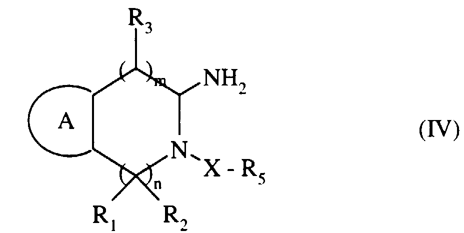 dans laquelle A, R₁, R₂, R₃, R₅, X, m et n ont la même signification que dans la formule (I), que l'on met en réaction :
- soit avec le carbonyldiimidazole et une hydroxylamine protégée,
pour conduire au composé de formule (I/e), cas particulier des composés de formule (I) : 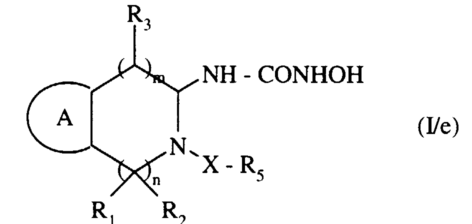 dans laquelle A, R₁, R₂, R₃, R₅, X, m et n ont la même signification que dans la formule (I),
- soit avec un bromoacétate, pour conduire au composé de formule (I/f), cas particulier des composés de formule (I) : 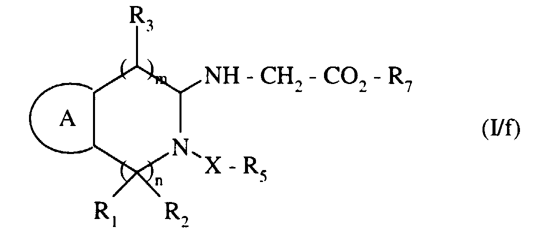 dans laquelle A, R₁, R₂, R₃, R₅, R₇, X, m et n ont la même signification que dans la formule (I),
• (d) dont on réduit l'acide en forme alcool correspondante, puis que l'on transforme en dérivé bromé par action de Pbr₃ dans l'éther,
pour conduire au composé de formule (V): 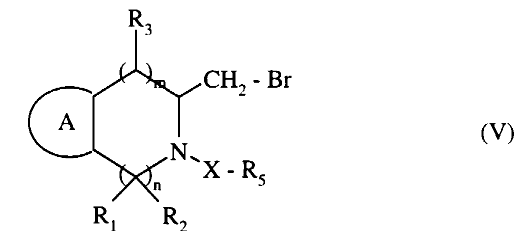 dans laquelle A, R₁, R₂, R₃, R₅, X, m et n ont la même signification que dans la formule (I), que l'on met en réaction :
- soit avec un malonate d'alkyle,
pour conduire, après saponification éventuelle, au composé de formule (I/g) : 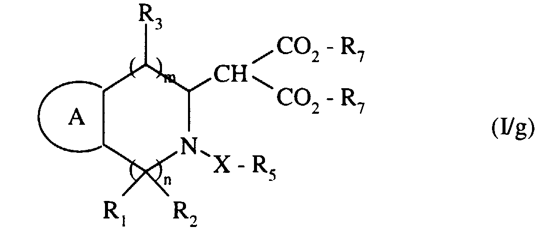 dans laquelle A, R₁, R₂, R₃, R₅, R₇, X, m et n ont la même signification que dans la formule (I),
- soit avec une glycine protégée,
pour conduire, après déprotection éventuelle, au composé de formule (I/h) : 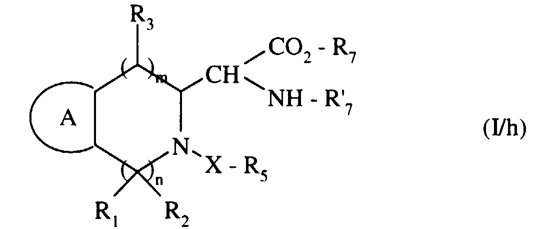 dans laquelle A, R₁, R₂, R₃, R₅, R₇, R'₇, X, m et n ont la même signification que dans la formule (I),
composé de formule (I/a) à (I/h), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

14. Compositions pharmaceutiques contenant comme principe actif au moins un composé selon l'une quelconque des revendications 1 à 12, seul ou en combinaison avec un ou plusieurs excipients ou véhicules inertes, non toxiques, pharmaceutiquement acceptables.

15. Compositions pharmaceutiques selon la revendication 14 contenant au moins un principe actif selon l'une quelconque des revendications 1 à 12 utiles comme inhibiteurs de métalloprotéases.
